(19) Europäisches Patentamt
European Patent Office
Office européen des brevets

(11) **EP 1 396 274 B2**

(12) **NEW EUROPEAN PATENT SPECIFICATION**
After opposition procedure

(45) Date of publication and mention
of the opposition decision:
**22.05.2013   Bulletin 2013/21**

(45) Mention of the grant of the patent:
**29.06.2005   Bulletin 2005/26**

(21) Application number: **02078806.3**

(22) Date of filing: **05.09.2002**

(51) Int Cl.:
**A61M 1/16** *(2006.01)*

(54) **Controller for a blood treatment equipment**

Steuerung für eine Blutbehandlungsvorrichtung

Contrôleur pour un appareil de traitement sanguin

(84) Designated Contracting States:
**AT BE BG CH CY CZ DE DK EE ES FI FR GB GR IE IT LI LU MC NL PT SE SK TR**

(43) Date of publication of application:
**10.03.2004   Bulletin 2004/11**

(73) Proprietor: **Gambro Lundia AB**
**22 643 Lund (SE)**

(72) Inventors:
• **Bene, Bernard**
  **69540 Irigny (FR)**
• **Vantard, Georges**
  **38090 Villefontaine (FR)**
• **Reitz, Carl W.**
  **4125 Riehen (CH)**

(74) Representative: **Ponzellini, Gianmarco**
**Gambro Intellectual Property Dept.**
**P.O. Box 98**
**41037 Mirandola (IT)**

(56) References cited:
**EP-A- 0 330 892      EP-A- 0 495 412**
**WO-A-98/55166      US-A- 4 244 787**
**US-A- 5 744 031**

## Description

FIELD OF INVENTION

This invention relates to a controller for a blood treatment equipment. The present invention relates also to a blood treatment equipment comprising said control apparatus.

More particularly, the invention is concerned with an apparatus, such s a programmable computer, capable of operating on a blood treatment equipment such as an hemodialysis equipment; the programmable controller is adapted to receive entries of prescribed and measured information and to generate one or more output signals in response thereto. In general the output signals are employed to control a variable operation performed by a dialysis equipment and hence automatically perform hemodialysis procedure control methods.

BACKGROUND OF THE INVENTION

As is for example explained in Gambro EP0330 892, it is of advantage to employ measured values of a patient's conditional values to control functional aspects of hemodialysis equipments. In this fashion, the hemodialysis equipment may be controlled dependently of specific treatment requirements of a patient. An important parameter includes actual clearance or dialysance values (expressed herein as D in ml/min). The value D is representative of the clearance of a blood solute and may be employed to determine a total dialysis dosage value $KT_t$ achieved after time $T_t$.

The approach presently followed is to obtain a measure of and to provide information related to a total dialysis dosage value delivered as time progresses during a hemodialysis treatment procedure. This measure and the information provided is essentially based on parameters which include:

a prescribed duration of the treatment procedure
the blood flow rate
the choice of the hemodialyser

A combination of above parameters is employed to obtain a measure of the total dialysis dosage value $KT_t$ delivered as an integral of mean measured instantaneous clearance values measured after determined time increments, the dialysance of the chosen dialyser (which is an in vitro clearance value) and the effective treatment time. The effective treatment time is the time during which diffusive (and generally also convective) transfer of blood solutes across a semi-permeable membrane of a hemodialyser takes place.

The above procedure basically enables a measure to be made of the KT value delivered to a patient during a hemodialysis treatment procedure. This procedure, however, suffers from a number of drawbacks. Specifically, such factors as blood flow rate and effective treatment time which are relevant to clearance are prone to change or are difficult to follow during a hemodialysis treatment procedure. Furthermore, the dialysance or clearance capacity of hemodialyser products can change significantly during a hemodialysis treatment procedure time. Present day hemodialysis monitoring equipment and hemodialysis procedure methods may comprise means for assessing or measuring dialysis dosages delivered to a patient over determined time increments, but no means are available for controlling the dialysis dosage value actually delivered to the patient within a prescribed treatment time.

It is an overall objective of the present invention to secure control over the actual total dialysis dosage delivered to a patient.

SUMMARY OF THE INVENTION

Hemodialysis monitoring equipment of the invention is associated with or comprises a programmable controller according to claim 1 adapted to receive entries of prescribed and measured information and to generate one or more control signals in response thereto. The controller is adapted to receive one or more entries of measured information measured during the course of a hemodialysis treatment procedure. The measured information is of such a nature that this can reflect one or more measures of dialysis dosages or dialysance values of a

Document EP 0 495 412 relates to the field of hemodialysis and the fact that many patients may not receive the prescribed dialysis dose Kt/V (urea), K being the clearance of urea by the dialyser, t the time and V the volume of distribution of urea.

This document describes a dialysis method providing dialysis dose by predicting a time end point for the dialysis. In this way, the dialysis dose of urea is selected by the physician, then the urea concentration is measured initially, and the end point prediction is based upon the preferred post-dialysis urea concentration and measured urea concentration values. Those data are compared, and if appropriate, dialysis should be terminated. In the event the comparison reveals that further dialysis is appropriate, a revised time end-point may be calculated.

The document WO98/55166 relates to a method and device for calculating the dialysis efficiency. The method uses urea concentration measured by a urea monitor in the effluent dialysate line for determining parameters of the dialysis as it progresses. These parameters are used for assessing the dialysis treatment on-line to determine the efficiency, the delivered dose, pre and post-treatment total urea masses in the body, urea generation rate, volume of distribution of urea in the body and further parameters. The dose of treatment can be calculated and when the desired dose has been obtained, the dialysis treatment is terminated.

So the two latest documents describe a method which measures one or more parameters during the treatment to calculate the effective dialysis dose in order to compare it with the prescribed dialysis dose. The only action taken after this comparison is to stop or continue with the treatment. hemodialyser employed in the hemodialysis treatment procedure. The hemodialysis monitoring equipment of the invention is characterised in that the controller is programmed to relate said one or more entries of said measured information received by the controller to both a prescribed dialysis dosage reference value and a prescribed weight loss reference value entered into the controller to obtain one or more inter-related values, and to generate one or more output control or command signals responsive to said one or more inter-related values to automatically control a fluid removal rate from the second compartment of the hemodialysis unit.

The nature of the operations performed by the hemodialysis monitoring equipment will depend on the nature of hemodialysis monitoring equipment. However, hemodialysis monitoring equipment of the state of the art will generally comprise means for discontinuing a hemodialysis treatment procedure after a prescribed treatment time, means for controlling a variable speed ultrafiltration pump in response to a prescribed weight loss and said prescribed treatment time, and means for setting the speed of blood and dialysate pumps. Some more modern hemodialysis monitoring equipment may comprise means for controlling the composition of dialysate employed in a hemodialysis treatment procedure. Also, some more modern hemodialysis monitoring equipment may comprise measuring means comprising conductivity sensors for measuring the conductivity of dialysate upstream and downstream of a hemodialyser product employed in the treatment procedure. The conductivity values (as intermittently influenced by intermittently introducing small boluses of higher or lower concentration solutions into the dialysis fluid upstream of the hemodialyser) may be employed to reflect instantaneous clearance or dialysance values at various points in time or after determined time increments, as is for example explained in the above-mentioned Gambro EP0330892. This type of hemodialysis monitoring equipment is preferably employed for providing the measured information employed in controlling variable operations performed by the hemodialysis monitoring equipment of the invention. However, measured information employed may alternatively be obtained by means of a urea sensor installed on the waste dialysate line which provides access to real time measure of urea clearance and urea mass transfer.

An important difference between the invention as described above and approaches followed in the past is that the hemodialysis treatment procedure time involved in the present invention need not be a prescribed time but may be a time which is dependent on achievement of a prescribed value. Thus, in accordance with the invention, the treatment time may be controlled by measured information which can be related to a measure of an effective clearance value of a substance (usually urea is the reference substance) measured after a determined time increment during a hemodialysis treatment procedure.

The controller associated with or comprised in the hemodialysis monitoring equipment of the invention may be programmed to generate output signals reflecting visual actual total dialysis dosages delivered after determined time intervals so that progress of a treatment procedure may be observed. However, more important, and in accordance with the invention, the controller may be programmed to compute that treatment procedure time which is required to achieve a prescribed total dialysis dosage value. In this case, the controller would be adapted to receive an entry of a prescribed total dialysis dosage value and to compute a hemodialysis treatment procedure time by relating entries of measured information to a prescribed total dialysis dosage value entered into the controller.

A computed hemodialysis treatment time which is a function of measured information of the above nature will in general be inclined to change during a treatment procedure and would therefore need to be corrected as new entries of measured information are received by the controller. The controller may be programmed to compute a total hemodialysis treatment procedure time by integrating over time clearance or dialysance values obtained from measured information measured after determined time increments to obtain a total of the dialysis dosage delivered over the effective treatment procedure time which has passed, and computing from this integrated value a total hemodialysis treatment procedure time at which a prescribed total dialysis dosage value would be achieved. Alternately, a remaining treatment time, which takes into account the total effective dialysis dosage already achieved after a determined time of treatment as compared to a prescribed total dialysis dosage may be computed. This remaining treatment time may once again need to be corrected as new entries of measured information are received by the controller. This procedure is an approach which is presently preferred.

Clearance values are influenced by ultrafiltration which leads to convective transfer of solutes in blood plasma across a semi-permeable membrane of a hemodialyser product into dialysis fluid. In practically all hemodialysis treatment procedures, ultrafiltration to achieve loss of excess fluid in the patient is required. The controller should therefore be adapted to include or account for the convective clearance which follows from ultrafiltration. Most preferably, therefore, the controller should be adapted to provide output information related to both the diffusive and convective clearance values or conveniently an integrated measure of these two values.

Ultrafiltration rates are set by setting the speed of a variable speed ultrafiltration pump. The speed of the pump is determined by a prescribed total weight loss value and, in earlier procedures, by a prescribed treatment time. In accordance with the present invention, the controller is adapted to receive an entry of a prescribed total weight loss value and to generate a rate control signal to control the rate of said variable speed ultrafiltration pump as a function of one or more entries of measured information of the nature described above and both a prescribed dialysis dosage reference value and a prescribed weight loss reference value entered into the controller.

By controlling the rate of the ultrafiltration pump as a function of measured information which can be related to diffusive clearance values and including or factoring in the convective clearance value as a function of the diffusive clearance value (which is equivalent to controlling the rate of the ultrafiltration pump as a function of the diffusive clearance value) it is possible to synchronise advents of achieving a prescribed total clearance or dialysis dosage delivered with achieving a prescribed total weight loss.

In line with an important consideration of the present invention to maintain a controlled relationship between diffusive clearance values and ultrafiltration rate, the controller may be adapted to compute and maintain a ratio of an ultrafiltration rate to a measured clearance or dialysis dosage value equal or proportional to a ratio of said prescribed total weight loss to said prescribed total clearance or dialysis dosage value.

The hemodialysis monitoring equipment of the invention may be associated with or comprise measuring means for obtaining measures of information of the nature described, and entry means for entering such measured information into the controller. The measuring means may comprise at least a downstream conductivity sensor for measuring the conductivity of dialysate downstream of a hemodialyser device. The measuring means may additionally comprise an upstream conductivity sensor for measuring the conductivity of dialysate upstream of a hemodialyser device.

Conveniently, the controller is adapted to generate an activating signal when or shortly before the computed hemodialysis treatment procedure time is reached. The activating signal may be employed to activate an alert device.

Hemodialysis procedure control methods disclosed correspond to measuring information, relating measured information to prescribed values as described above in relation to the hemodialysis monitoring equipment of the invention, making the computations and performing the control functions as similarly described.

## DESCRIPTION OF THE DRAWINGS

The invention will be described with reference to the accompanying exemplary drawing tables, wherein:

Fig 1 is a schematic drawing of hemodialysis equipment associated with a controller.

Fig 2 is a flow diagram showing various alternative routes which may be followed dependently of measured and prescribed values entered into the controller.

Fig 3 schematically shows a display screen that would be associated with the controller referred to in Fig 1.

Fig 4 is a flow diagram explaining functions controlled by the controller to set ultrafiltration rate.

Fig 5 is a flow diagram of the functions controlled by the controller when a computed remaining hemodialysis treatment time is less than, for example, 15 min.

Fig 6 is a flow diagram of what occurs if a computed total hemodialysis treatment time exceeds a prescribed maximum treatment time and the routes followed if staff intervention is positive or negative.

Fig 7 is a flow diagram explaining what attending staff would do and what functions would then be controlled by the controller to set ultrafiltration rate.

## DETAILED DESCRIPTION

A specific and presently preferred example of blood treatment equipment, associated with or comprising a controller according to the invention, is described below with reference to the accompanying schematic drawing and flow diagrams in which and where employed in the claims, the symbols below will have the meanings identified as follows:

| | |
|---|---|
| $Tt$ = | elapsed dialysis treatment time |
| $Tt_r$ = | remaining dialysis treatment time |
| $Tm$ = | maximum dialysis treatment time |
| $Da$ = | average dialysance |
| $DTt$ = | dialysance measured at time $Tt$ |
| $KT$ = | dialysis dosage value |
| $KTp$ = | prescribed dialysis dosage value |
| $KTt$ = | integrated dialysis dosage value at time $Tt$ |
| $WL$ = | weight loss |
| $WLp$ = | prescribed weight loss |
| $WLTt$ = | weight loss at time $Tt$ |
| $UF$ = | ultrafiltration rate |
| $UFTt$ = | ultrafiltration rate at time $Tt$ |

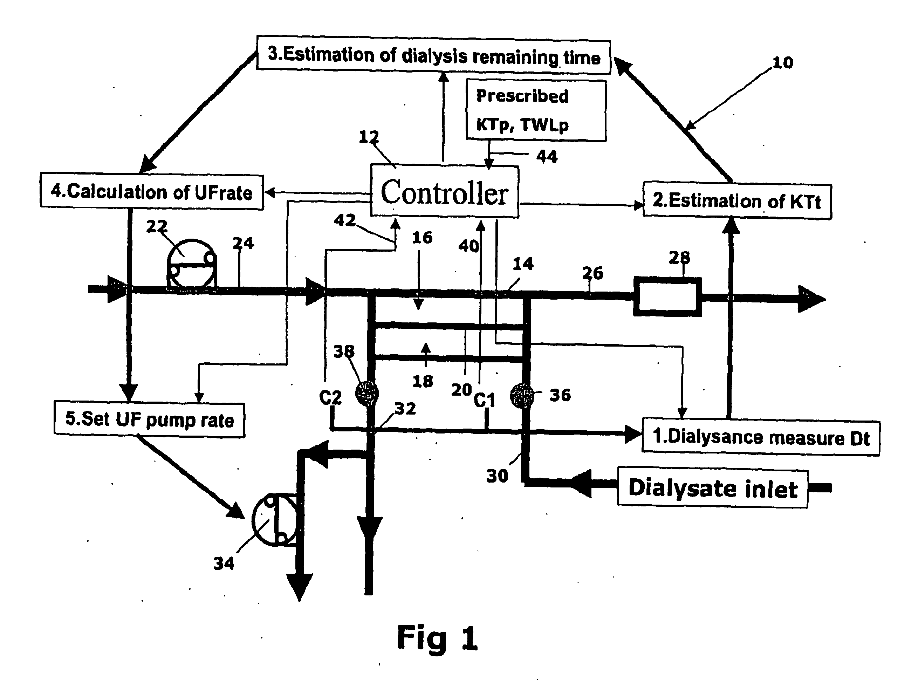

Referring to the Figure 1 schematic drawing, reference numeral 10 refers generally to a blood treatment equipment, such as for instance hemodialysis equipment, comprising or associated with a controller 12, for instance a programmable controller. The equipment is shown to be connected to a blood treatment unit 14, such as a hemodialyser, comprising a blood compartment 16 and a dialysate compartment 18 divided by a semi-permeable membrane 20. A blood pump 22 is provided upstream of the hemodialyzer for pumping blood from a patient along blood arterial line 24 into the blood compartment 16 and out from the blood compartment along blood venous line 26 to drip chamber 28 and back to the patient.

Dialysate is conveyed into the dialysate compartment 18 along dialysate inlet line 30 and out from the dialysate compartment along dialysate outlet line 32 in a direction counter-current to blood flow in the hemodialyzer 14. A variable speed ultrafiltration pump 34 is provided for pumping ultrafiltrate from blood comprised in blood compartment 16 across the semi-permeable membrane into the dialysate chamber 18 and out from the dialysate outlet line 32. The flow rate of dialysate into and out of the dialysate compartment 18 is controlled by conventional means, e.g. by means of flow meters (not shown) located upstream and downstream of the hemodialyzer product or by controlling volumes of dialysate delivered to and withdrawn from the dialysate compartment 18. An infusion line may be provided with (not shown in the appended drawing tables) for injecting replacement fluid in the arterial and/or in the venous line 24,26.

The equipment 10 is able to perform different treatments such as:

- conventional hemodialysis, HD, where no infusion is present and dialysis liquid circulates in the second compartment of the dialyzer;
- hemofiltration, HF, where no dialysis liquid is present while solutes and plasma water are pumped through line 32 and substitution fluid is infused in the extracorporeal circuit or directly into the patient;
- hemodiafiltration, HDF, which is a combination of HD and HF.

It is of relevance determining, during the treatment, one or more parameters indicative of the progress of the treatment itself in order to intervene actively on the equipment 10 in view of the desired therapeutic objectives. The controller 12 is therefore designed to calculate a significant parameter indicative of the progress of an extracorporeal blood treatment carried out by equipment 10. Indicative parameters that can give an indication of the actual progress of a dialysis treatment are one or more of the following:

- the concentration Cb of a substance (sodium for instance) in the blood of a patient undergoing a dialysis treatment;
- the dialysis dose KTt after a time Tt.

European patents number EP 0547025B1 and EP 0658352B1, describe alternative ways for in vivo determination of the actual dialysance, blood sodium concentration and dialysis dose. Note that any method able to determine one or more of the above significant parameters can be used for the purpose of the present invention.

Referring by way of non-limiting example to a first known method for determining the concentration of a substance in blood and/or the actual dialysance for said substance (described in detail in EP 0547025B1), at least two liquids differing for their respective concentration of said specific substance are sequentially circulated through the dialysate compartment 18.

The first liquid can be the dialysis liquid at its normal prescribed value of concentration for the substance and the second liquid can be obtained by introducing a step in the concentration of said substance at the dialyzer 14 inlet.

Then the conductivity or concentration of the substance are measured for the first ad second liquid both upstream and downstream of the dialyzer. Note that the upstream measurements can be substituted by set reference values.

Notice in this respect that if the substance is a ionic substance, then the concentration of the substance influences the conductivity of the dialysis liquid; in particular considering that conductivity is largely influenced by the concentration of sodium ions, than measure/calculation of conductivity values gives an indication of sodium concentration in blood and in the dialysis liquid. As conductivity sensors are much more convenient and easy to use than ion selective sensors for directly detecting the concentration of an electrolyte in a liquid flow, conductivity measurements are preferably used.

Referring to figure 1, conductivity or sensors 36 and 38 are provided which are respectively located for measuring the conductivity of dialysate flowing to dialysate compartment 18 along dialysate inlet line 30. In detail, conductivity sensor 36 provides upstream dialysate conductivity measures $C_{1in}$, $C_{2in}$ relating to the conductivity of the first and second liquid upstream the dialyzer, while conductivity sensor 38 measures the conductivities $C_{1out}$, $C_{2out}$ of the first and second dialysis liquid flowing from dialysate compartment 18 along dialysate outlet line 32. The measures of conductivity (as intermittently influenced by intermittently introducing small boluses of higher or lower concentration dialysate solutions into the dialysate inlet line 30) may be employed to reflect instantaneous dialysance values at any point in time Tt during a hemodialysis treatment procedure or after determined time increments so that a dialysis dosage delivered may be determined.

As a final step the concentration of the substance in blood and/or the the actual dialysance for said substance can be obtained from the measured conductivities or concentrations of the substance in the first and second liquid.

In detail the following formulas can be used (no ultrafiltration and neglecting the so-called Donnan effect):

$$Cdout = Cdin + (Cbin - Cdin) * DTt/Qd$$

Where

Cdout = conductivity (or sodium concentration) of used dialysis liquid downstream dialyzer;

Cdin = conductivity (or sodium concentration) of fresh dialysis liquid upstream dialyzer;

Cbin = conductivity (or sodium concentration) in untreated blood;

DTt = dialysance for sodium/conductivity measured at time Tt;

Qd = dialysis liquid flow.

KTt = QTt if ultrafiltration is set to 0.

The above equation can be written for the two dialysis liquids circulated through the dialyzer so that the two unknowns DTt and Cbin can be determined.

Referring again to the drawings, the periodically measured conductivity values $Cd_{1in}$, $Cd_{2in}$ and $Cd_{1out}$, $Cd_{2out}$ (1 and 2 referring to the first and second liquid respectively) are entered into the controller via lines 40 and 42. The controller can then calculate DTt and Cbin by using for instance the above described method and also estimate the clearance K and the dialysis dose KTt/V, where Tt is the elapsed treatment time and V is the total volume of water for the patient.

Prescribed dialysis dosage reference values KTp and prescribed total weight loss reference value LWp (TWLp) are similarly entered into the controller via entry means 44 of any kind: a data reader, a keyboard, a remote station.

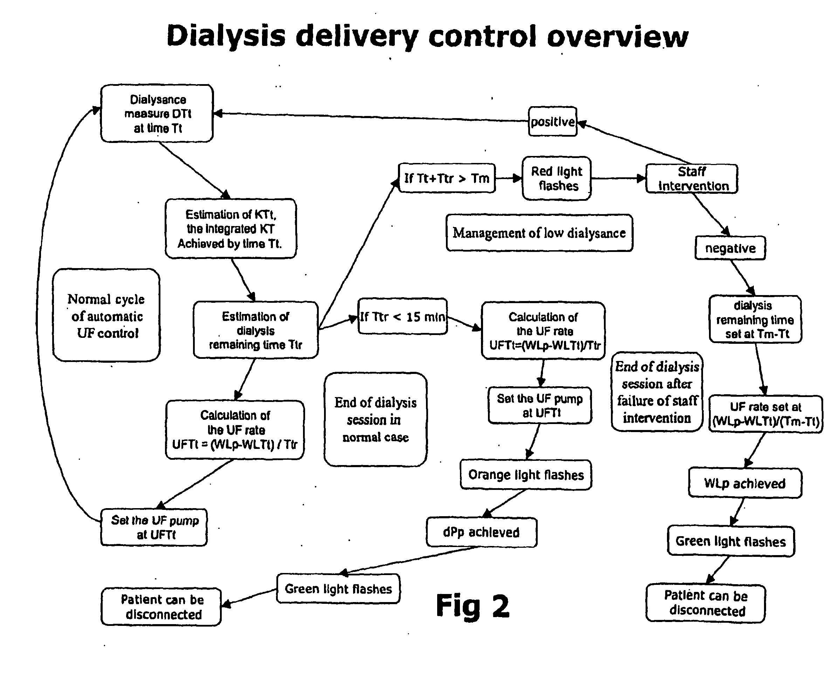

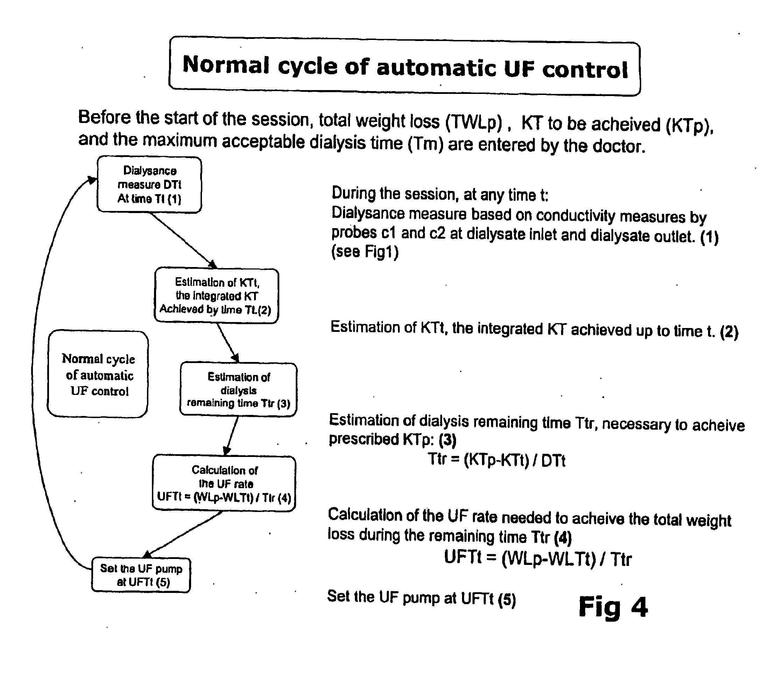

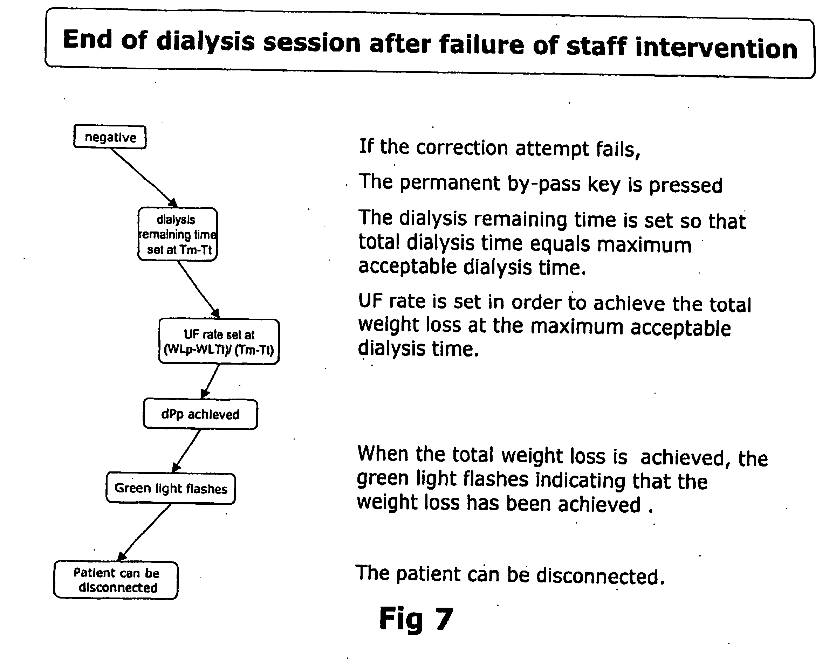

The controller is programmed to perform the calculations or estimations shown, in accordance with estimation or calculation procedures described in detail hereafter or as described in relation to Figures 2, 4 and 7 of the accompanying flow diagrams.

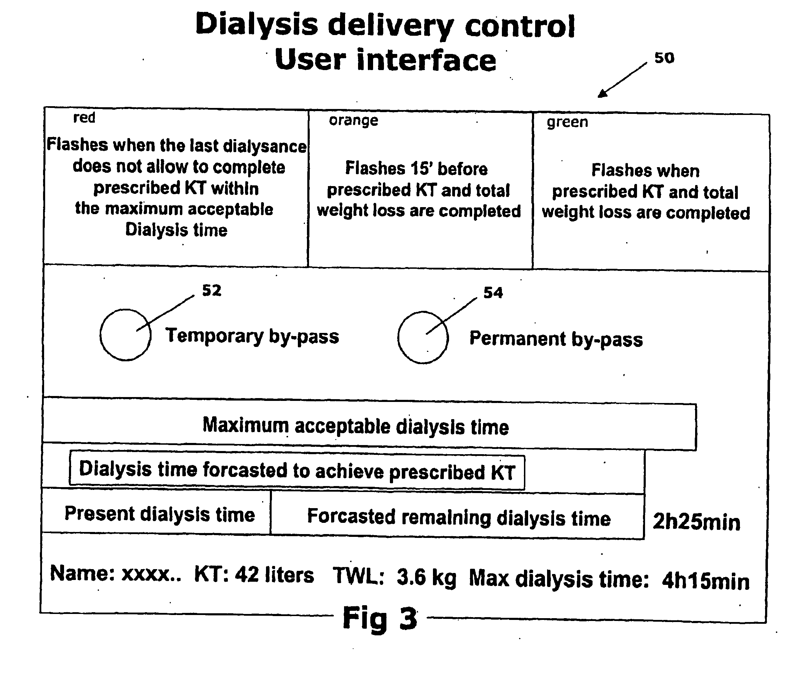

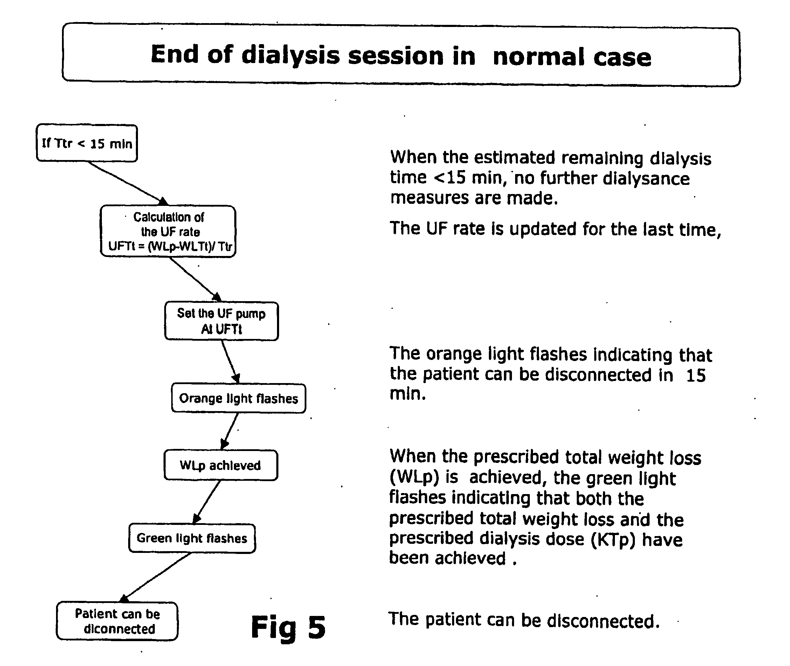

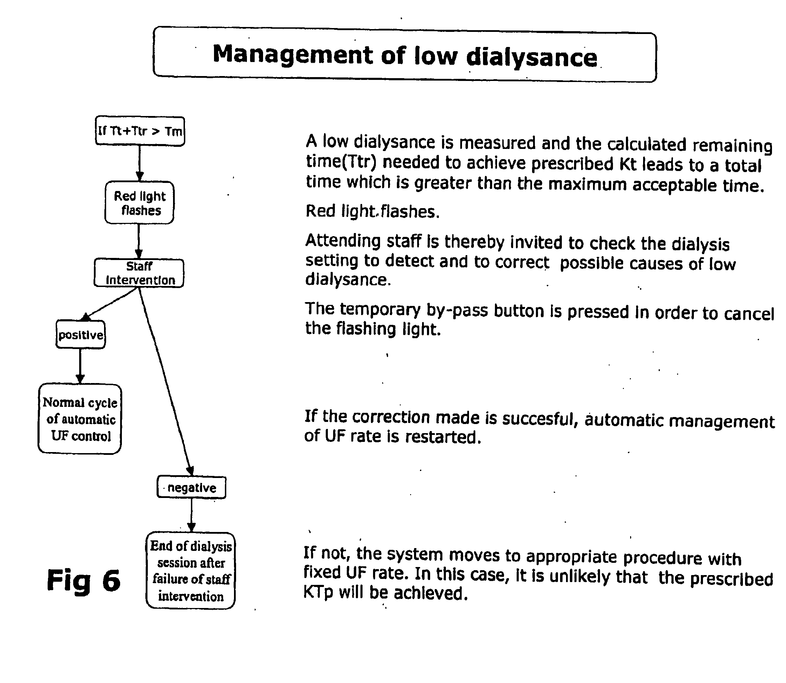

Figure 3 shows a display screen 50 which would be associated with the controller 12 and would provide visual indications of events as shown and information reflected by the controller based on measured information measured during the course of a hemodialysis treatment procedure, as is further explained in conjunction with the flow diagrams of Figures 5, 6 and 7. The display screen also comprises temporary and permanent by-pass buttons 52 and 54 which would be depressed by attending staff if certain events occur, e.g. as explained in relation to the flow diagram of Figure 6. The permanent by-pass button would for example be depressed if a new dialysis treatment setting is to be initiated or if a treatment procedure is to be discontinued.

Going in further detail, it should be borne in mind that it is an overall objective of the present invention to secure control over the actual total dialysis dosage delivered to a patient; this control can for example be achieved, in accordance with the invention, by computing a hemodialysis treatment procedure time as a function of calculated values related to one or more of the above identified significant parameters (such as a dialysis dosage value reached after at treatment time t); a basic component of such computation would comprise a determination of a treatment time as a function of such one or more calculated values. Thus, in this example, a computed total effective treatment time would need to be a function of one or more values $KTt_1$, $KTt_2$, $KTt_3$, ---, KTtn, calculated in vivo using any known method after determined time increments $\Delta t$ = say 5 min. For practical reasons it may only be possible to obtain a first measured value after about say 15 min of effective treatment time. Presuming this to be the case, a reasonably accurate assessment of an initial clearance or dosage value KTti which has been achieved during said 15 min initial treatment time can be obtained by assuming that the measured clearance value or dosage delivered, for example after a 5 min interval, will substantially equate with the clearance value delivered over the same time period before the first measurement is made.

Successive measurements of clearance values would generally be at least fractionally different from one another in that these values are dependent on changes (usually lowering) of the clearance capacity of the dialyser product during a treatment procedure, changes of blood rate, possible recirculation of treated blood, dialysis liquid flow rate, ultrafiltration rate and other changes.

Measurements of clearance values would only be made during effective treatment times, i.e. while blood and dialysis liquid are flowing through the hemodialyser product. The controller is accordingly be programmed to initiate measurements only during effective treatment times and similarly only compute or integrate effective treatment times to arrive at a computed hemodialysis treatment procedure time during effective treatment times.

It would be possible to compute a hemodialysis treatment procedure time as a function of measured values in various fashions, e.g. by reference of the difference between successive total dialysis dosage values to a reference difference value and to compute an increase or decrease in the treatment time proportional to deviations from the reference difference value. Such a procedure could for example be realised more readily if a standardised total clearance or dialysis dosage value is to be achieved. However, a simpler, more adaptable and reliable procedure is to compare measured values with a prescribed total dialysis dosage value specifically prescribed for the particular patient condition. In this fashion the dialysis treatment procedure time at which the prescribed total dialysis dosage value will be reached can be computed. Exemplary of this procedure is the following:

Before initiating a hemodialysis treatment procedure,

• The total clearance value KT to be achieved is prescribed (KTp).

Then during the treatment,

- The effective total dialysis dosage value which has been achieved by a determined effective treatment timeT t is computed (KTt)
- The remaining treatment procedure time (Tt$_r$) is computed e.g. based on a computation of the ratio of the difference between the prescribed total clearance value KTp and the computed effective total dialysis dosage achieved by time Tt (KTt) to the instantaneous dialysance value measured at time Tt (DTt) i.e.

$$Tt_r = \frac{(KTp - KTt)}{DTt}$$

As mentioned, practically all treatment procedures involve ultrafiltration to achieve a prescribed total weight loss (WLp or TWLp) during the effective treatment procedure time. Since it is most desirable to complete a hemodialysis treatment procedure in as short a time as is possible, the ultrafiltration rate controlled by a variable speed ultrafiltration pump should be set to achieve the prescribed total weight loss WLp within the effective time of the treatment procedure. Accordingly the speed of the ultrafiltration pump may be set to achieve the prescribed total weight loss at a point in time which is earlier, say 20 min earlier, than the time by which the prescribed total clearance KTp might be achieved. Thus, the total time that the ultrafiltration pump is operative may be somewhat less than the effective treatment time during which diffusion of solutes from blood into dialysis fluid, i.e. diffusive clearance, takes place. However, also for the reason that ultrafiltration influences clearance values, it is preferable that the ultrafiltration pump is operative over the same period of time as diffusive clearance of solutes from blood is taking place. With this preference in mind, and in accordance with the invention, the setting of the speed of the ultrafiltration pump is preferably controlled by the controller in such a fashion that a prescribed total weight loss WLp is achieved at the same time as the prescribed total dialysis dosage value KTp is achieved.

Synchronising an achievement of a prescribed total weight loss WLp with an achievement of a prescribed total dialysis dosage value KTp can be automatically secured by suitable adaption of the controller, for example by relating an actual measured total ultrafiltration volume achieved by time Tt to the prescribed total weight loss WLp, and controlling the rate of the ultrafiltration pump in response to the compared values and the estimated remaining treatment procedure time Tt$_r$ referred to above in connection with dialysis dosage values to be achieved. The estimated remaining treatment procedure time is a function of a measured instantaneous dialysance value DTt measured at time Tt, so that the setting of the rate of the ultrafiltration pump will similarly be a function of this instantaneous measured dialysance value. Thus, the ultrafiltration rate at time Tt (UFTt) is set to be equal to the prescribed total weight loss WLp less the measured weight loss at time Tt, i.e. WLTt, divided by the estimated remaining treatment time Tt$_r$, i.e.

$$UFTt = \frac{(WLp - WLTt)}{Tt}$$

The total treatment time T or remaining treatment time Tt$_r$ at time Tt is regularly recalculated and updated on the basis of the last or most recent instantaneous measured clearance or dialysance value DTt. Thus, any such changes in parameters which take place during a hemodialysis treatment procedure which may influence the dialysance or clearance of a hemodialyser product, such as blood flow rate, dialysis fluid flow rate, alterations in the permeability of the semi-permeable membrane of the hemodialyser product, will automatically be accounted for each time the treatment time is recalculated. This procedure of the invention accordingly provides a reliable means for securing a measure of the treatment time required to secure the prescribed dialysis dosage value KTp.

As mentioned, the above procedure may also involve corresponding control of the rate of ultrafiltration in such a fashion that the ultrafiltration rate is also an indirect function of instantaneous measured dialysance values by virtue of treatment times being a function of such measured values.

Measured dialysance values will generally include a measure of convective clearance values obtained by ultrafiltration. An alternative approach to the invention is to maintain a ratio of ultrafiltration rate UF to average dialysance Da proportional or equal to a ratio of a prescribed total weight loss WLp to a prescribed total clearance or dialysis dosage value KTp, i.e.

$$\frac{UF}{Da} = \frac{TWLp}{KTp}$$

Since WLp and KTp are known values $\frac{WLp}{KTp} =$ a known value R. Thus, at any dialysance measure at the time t, i.e. DT$_t$, the ultrafiltration rate at time Tt, i.e. UFTt would be set at DTt • R, i.e. UFTt = DTt • R. In this procedure, the effective time of the hemodialysis treatment procedure can be ended when the prescribed total weight loss WLp has been achieved. In this case, the prescribed total weight loss can be employed as the overriding factor in the computation of the hemodialysis treatment procedure time rather than the prescribed total clearance or dialysis dosage value KTp. However, since the above-mentioned known value R is a ratio of the prescribed total weight loss WLp and the prescribed total clearance or dialysis dosage value KTp, the KTp value will also be at least substantially achieved at the time that the prescribed total weight loss WLp is achieved.

The above alternative approach to the invention may provide a convenient approach in that the ultrafiltration pump may be set at a known speed at the commencement of a hemodialysis treatment procedure and thereafter altered in accordance with the above UFTt = DTt • R equation. As already mentioned, a first dialysance or clearance measurement can generally only be made after an initial elapse of time of say 15 min, so that only an estimation based on later measurements of what dialysis dosage has been delivered during the first 15 min can be made.

**Claims**

1. A controller (12) for a blood treatment equipment (10), said equipment comprising at least a treatment unit (14) including a semipermeable membrane separating the treatment unit in a first compartment (16) for the circulation of blood and in a second compartment (18) for the circulation a of a treatment liquid, the controller (12) being adapted to:

   - receive one or more entries of measured information measured during the course of a treatment procedure, said measured information being one chosen in the group comprising conductivity of the treatment liquid downstream the treatment unit (14); concentration of a substance in the treatment liquid downstream the treatment unit (14);
   - calculate from said measured information at least a significant parameter indicative of the progress of an extracorporeal blood treatment carried out by the equipment (10),
   - compare said calculated significant parameter to a prescribed reference value for the same parameter,
   - generate at least one output control signal responsive to said comparison,

   **wherein**
   said output control signal is generated for automatically controlling a fluid removal rate from said second compartment (18);
   the significant parameter indicative of the progress is one chosen in the group comprising:

   - the concentration Cb of a substance in the blood of a patient undergoing a treatment;
   - the dialysis dose KTt after a time Tt.

2. Controller (12) according to claim 1 programmed to relate said one or more significant parameters to both a prescribed dialysis dosage reference value and a prescribed weight loss reference value entered into the controller (12) to obtain one or more inter-related values, and to generate at least one or more output control signals responsive to said one or more inter-related values to automatically control one or more variable operations performed by the equipment (10).

3. Controller (12) according to claim 2, in which said one or more inter-related values comprises a multiplied relationship between said one or more entries of said measured information and a ratio of a difference between said prescribed dialysis dosage value and a measure of a delivered dialysis dosage to a difference between said prescribed weight loss value and an achieved weight loss, or the inverse of such ratio as respectively represented by

$$\frac{(WLp - WLTt) \cdot DTt}{(KTp - KTt)},$$

or the inverse of such ratios, wherein the symbols have the meanings identified herein.

4. Controller (12) according to claim 1, in which the controller (12) is programmed to compute a total delivered dialysis dosage in response to measured information received by the controller (12), and to generate an output command signal when said measured information received by the controller (12) reflects a measure of a total delivered dialysis dosage which approximates or equates with said prescribed dialysis dosage value.

5. Controller (12) according to claim 1, in which the controller (12) is programmed to compute a total delivered dialysis dosage at one or more determined time increments during a treatment procedure in response to measured information received by the controller at said one or more determined time increments, and to generate an output command signal when said measured information received by the controller (12) reflects a measure of a total delivered dialysis dosage which approximates or equates with said prescribed dialysis dosage value.

6. Controller (12) according to claim 1, in which the controller (12) is programmed to compute a hemodialysis treatment procedure time or remaining hemodialysis treatment procedure time by relating a computation of a delivered dialysis dosage reflected by an entry of measured information received by the controller (12) after a determined time increment during a hemodialysis treatment procedure to said prescribed dialysis dosage value.

7. Controller (12) according to claim 6, in which the controller is programmed to:

determine a plurality of values of said significant parameter, preferably including the dialysis dosage, after a plurality of determined time increments,

integrate said plurality of values over said plurality of time increments to reflect an integrated measure of a total value of said significant parameter delivered, preferably the total delivered dialysis dosage, as related to an integral or total of said plurality of determined time increments.

8. Controller (12) according to claim 6, in which the controller (12) is programmed to compute a remaining treatment procedure time by subtracting said measure of a delivered dialysis dosage from said prescribed dialysis dosage value and dividing the resulting difference by an average dialysance value represented by said delivered dialysis dosage divided by said determined time increment.

9. Controller (12) according to claim 6, in which the controller (12) is programmed to compute a remaining treatment procedure time by subtracting said measure of a delivered dialysis dosage from said prescribed dialysis dosage value and dividing the resulting difference by an instantaneous dialysance value measured at the end of said determined time increment, as represented by (KTp - KTt)/DTt.

10. Controller according to claim 1, wherein the equipment includes a variable speed ultrafiltration pump (34), said one or more output

control signals responsive to said one or more inter-related values generated by the controller (12) are employed to automatically control the speed of said variable speed ultrafiltration pump (22).

11. Controller (12) according to claim 9, in which said one or more inter-related values comprises a multiplied relationship between said one or more entries of said measured information and a ratio of a difference between said prescribed dialysis dosage value and a measure of a delivered dialysis dosage to a difference between said prescribed weight loss value and an achieved weight loss, or the inverse of such ratio as respectively represented by

$$\frac{(WLp - WLTt) \cdot DTt}{(KTp - KTt)},$$

or the inverse of such ratios, wherein the symbols have the meanings identified herein.

12. Controller (12) according to claim 11, in which the controller (12) is programmed to generate a control signal responsive to said multiplied relationship to automatically control the speed of said variable speed ultrafiltration pump (22) to maintain said ratio or inverse thereof, whereby the entered prescribed total weight loss reference value may be achieved substantially simultaneously with delivery of the entered prescribed dialysis dosage reference value.

13. Controller (12) according to claim 10, in which the controller (12) is programmed to reflect a measure of a total weight loss achieved as a function of the speed of the variable speed ultrafiltration pump (22) and a determined time increment during a hemodialysis treatment procedure.

14. Controller (12) according to claim 6, the equipment (10) including a variable speed ultrafiltration pump (22), in which the controller (12) is programmed to generate a control signal to automatically control the speed of said variable speed ultrafiltration pump (22) as a function said computed treatment procedure time or remaining treatment procedure time and said prescribed weight loss reference value entered into the controller (12).

15. Blood treatment equipment (10) comprising at least a treatment unit (14) including a semipermeable membrane (20) separating the treatment unit (14) in a first compartment (16) for the circulation of blood and in a second compartment (18) for the circulation of a treatment liquid, and a controller (12) according to anyone of the preceding claims.

16. Equipment (10) according to claim 15 comprising measuring means for obtaining one or more measures of information during the course of a hemodialysis procedure, which information can reflect one or more measures of dialysis dosage values or dialysance values of said treatment unit (14), this latter including a hemodialyser, the equipment also including entry means for entering such measured information into the controller.

17. Equipment (10) according to claim 16, in which the measuring means comprises a downstream conductivity sensor (38) for measuring the conductivity of dialysate in a dialysate line (32) downstream of the treatment unit (14).

18. Equipment (10) according to claim 16, in which the measuring means additionally comprises an upstream conductivity sensor (36) for measuring the conductivity of dialysate in a dialysate line (30) upstream of the treatment unit (14).

19. Equipment (10) according to claim 16, in which the controller (12) is programmed to reflect one or more dialysance values of said hemodialyser (14) at one or more determined time increments during a hemodialysis treatment procedure and to compute a hemodialysis treatment procedure time or remaining hemodialysis treatment procedure time by relating such dialysance values and determined time increments to said prescribed dialysis dosage reference value entered into the controller (12).

20. Equipment (10) according to claim 19, comprising a variable speed ultrafiltration pump (34), in which the controller (12) is programmed to generate a control signal to automatically control the speed of the variable speed ultrafiltration pump (34) as a function of said computed hemodialysis treatment procedure time or remaining hemodialysis treatment procedure time and said prescribed weight loss reference value entered into the controller (12).

21. Equipment (10) according to claim 19, in which a prescribed maximum hemodialysis treatment procedure time is entered into the controller and in which the controller is programmed to compare said computed hemodialysis treatment time or remaining hemodialysis treatment time with said prescribed maximum hemodialysis treatment procedure time and, if a total computed hemodialysis treatment time exceeds said prescribed maximum treatment time, to generate an output control signal to control the speed of the ultrafiltration pump (34) to achieve said prescribed weight loss reference value when said prescribed maximum hemodialysis treatment time is reached.

22. Equipment (10) according to claim 17, in which the controller (12) is associated with an alert device, in which a prescribed maximum hemodialysis treatment procedure time is entered into the controller (12) and in which the controller (12) is programmed to compare said computed hemodialysis treatment procedure time or remaining hemodialysis treatment time with said prescribed maximum hemodialysis treatment procedure time and, if a total computed hemodialysis treatment time exceeds said entered prescribed maximum treatment time, to generate a command signal to activate said alert device.

23. Equipment (10) according to claim 15, in which the controller (12) is associated with a display screen (50) adapted to display an output signal from the controller (12) reflecting said measures of delivered dialysis dosages or dialysance values of a hemodialyser reflected by said measured information.

24. Equipment (10) according to claim 15, in which the controller (12) is associated with a display screen (50) adapted to display an output signal from the controller (12) reflecting said total delivered dialysis dosage reflected in response to measured information received by the controller (12).

25. Equipment (10) according to claim 15, in which the controller (12) is associated with a display screen (50) adapted to display an output signal from the controller (12) reflecting said hemodialysis treatment time or remaining hemodialysis treatment time computed by the controller (12).

26. Equipment (10) according to claim 15, in which the controller (12) is associated with a display screen (50) adapted to display said prescribed dialysis dosage reference value entered into the controller (12).

27. Equipment (10) according to claim 15, in which the controller is associated with a display screen (50) adapted to display said prescribed weight loss reference value entered into the controller (12).

28. Equipment (10) according to claim 15, in which the controller (12) is associated with a display screen (50) adapted to display said measure of a total weight loss achieved.

29. Equipment (10) according to claim 15, in which the controller (12) is associated with a display screen (50) adapted to display said prescribed maximum hemodialysis treatment procedure time entered into the controller (12).

30. Program storage means including a program for a programmable controller (12) according to one of the preceding claims, the program when run by the controller (12) rendering the controller (12) adapted to execute a method of automatically controlling one or more variable operations performed by hemodialysis monitoring equipment (10) comprising at least a treatment unit (14) including a semipermeable membrane separating the treatment unit in a first compartment (16) for the circulation of blood and in a second compartment (18) for the circulation of a treatment liquid, said equipment (10) being associated with or comprising the programmable controller (12), said method comprising the steps of:

- receiving one or more entries of measured information measured during the course of a treatment procedure, said measured information being one chosen in the group comprising conductivity of the treatment liquid downstream the dialyzer (14); concentration of a substance in the treatment liquid downstream the dialyzer (14);
- calculating from said measured information at least a significant parameter indicative of the progress of an extracorporeal blood treatment carried out by the equipment (10),
- comparing said calculated significant parameter to a prescribed reference value for the same parameter,

- generating at least one output control signal responsive to said comparison,

- automatically controlling from said output control signal a fluid removal rate from said second compartment (18);

wherein the significant parameter indicative of the progress is one chosen in the group comprising:

- the concentration Cb of a substance in the blood of a patient undergoing a treatment;
- the dialysis dose KTt after a time Tt.

31. Program storage means according to claim 30 comprising an optical data carrier and/or a magnetic data carrier and or a volatile memory support.

**Patentansprüche**

1. Steuerung (12) für eine Blutbehandlungsvorrichtung (10), wobei die Vorrichtung mindestens eine Behandlungseinheit (14) umfasst, die eine semipermeablen Membran beinhaltet, die Behandlungseinheit in ein erstes Abteil (16) zur Blutzirkulation und ein zweites Abteil (18) zur Zirkulation einer Behandlungsflüssigkeit trennt, wobei die Steuerung (12) angepasst ist zum:

- Empfangen einer oder mehreren Eingaben von während einer Behandlung gemessenen Messdaten, wobei die Messdaten ausgewählt werden aus der Gruppe umfassend die Leitfähigkeit der Behandlungsflüssigkeit abwärts von der Behandlungseinheit (14); die Konzentration eines Stoffs in der Behandlungsflüssigkeit abwärts von der Behandlungseinheit (14);

- Berechnen, ausgehend von den Messdaten, mindestens eines bedeutenden Parameters, der den Verlauf einer extrakorporalen, durch die Vorrichtung (10) durchgeführten Blutbehandlung angibt,

- Vergleichen des bedeutenden Parameters mit einem vorgegebenen Bezugswert für denselben Parameter,

- Erzeugen mindestens eines Ausgangssteuersignals als Reaktion auf den Vergleich,

wobei

das Ausgangssteuersignal zur automatischen Steuerung einer Fluidentfernungsrate aus dem zweiten Abteil (18) erzeugt wird;

wobei der bedeutende, den Verlauf angebende Parameter ausgewählt wird aus der Gruppe umfassend:

- die Konzentration Cb eines Stoffs im Blut eines Patienten, der einer Behandlung unterworfen ist;

- die Dialysedosis KTt nach einer Zeit Tt.

2. Steuerung (12) nach Anspruch 1, programmiert zum Verbinden des einen oder mehrerer bedeutender Parameter sowohl mit einem vorgegebenen Dialysedosisbezugswert als auch mit einem vorgegebenen Gewichtsverlustbezugswert, die in die Steuerung (12) eingegeben werden, um einen oder mehrere miteinander verbundenen Werte zu erhalten, und zum Erzeugen mindestens eines oder mehrerer Ausgangssteuersignale in Reaktion zum einen oder mehrerer in Wechselbeziehung stehender Werte, um eine oder mehrere, durch die Vorrichtung (10) durchgeführte, regelbaren Operationen automatisch zu steuern.

3. Steuerung (12) nach Anspruch 2, in der die einen oder mehrere in Wechselbeziehung stehende Werte eine multiplizierte Relation zwischen der einen oder mehrerer Eingaben der Messdaten und einem Verhältnis eines Unterschieds zwischen dem vorgegebenen Dialysedosisbezugswert und einer Messung einer gelieferten Dialysedosis zu einem Unterschied zwischen dem vorgegebenen Gewichtsverlustwert und einem erreichten Gewichtsverlust, oder dem Kehrwert eines solchen Verhältnisses, wie es von:

$$\frac{(WLp - WLTt) \cdot DTt}{(KTp - KTt)},$$

dargestellt ist, oder dem Kehrwert solcher Verhältnisse umfasst, wobei die Symbole die hier angegebene Bedeutung haben.

4. Steuerung (12) nach Anspruch 1, in der die Steuerung (12) programmiert ist zum Berechnen einer gelieferten Gesamtdialysedosierung in Reaktion auf die von der Steuerung (12) empfangenen Messdaten, und zum Erzeugen eines Ausgangssteuersignals, wenn die von der Steuerung (12) empfangenen Messdaten eine Messung einer gelieferten Gesamtdialysedosierung darstellen, die dem vorgegebenen Dialysedosierungswert nahekommt oder entspricht.

5. Steuerung (12) nach Anspruch 1, in der die Steuerung (12) programmiert ist zum Berechnen einer gelieferten Gesamtdialysedosierung zu einem oder mehreren bestimmten Zeitinkrementen während einer Behandlung in Reaktion auf die von der Steuerung empfangenen Messdaten zu den einen oder mehreren bestimmten Zeitinkrementen, und zum Erzeugen eines Ausgangssteuersignals, wenn die von der Steuerung (12) empfangenen Messdaten eine Messung einer gelieferten Gesamtdialysedosierung darstellen, die dem vorgegebenen Dialysedosierungswert nahekommt oder entspricht.

6. Steuerung (12) nach Anspruch 1, in der die Steuerung (12) programmiert ist zum Berechnen einer Hämodialysebehandlungszeit oder einer restlichen Hämodialysebehandlungszeit durch in Verhältnis Setzen einer Berechnung einer gelieferten Dialysedosierung, die von einer Eingabe der von der Steuerung (12) empfangenen Messdaten dargestellt ist, nach einem bestimmten Zeitinkrement während einer Hämodialysebehandlung mit dem vorgegebenen Dialysedosierungswert.

7. Steuerung (12) nach Anspruch 6, in der die Steuerung programmiert ist zum:

   Bestimmen einer Vielzahl von Werten des bedeutenden Parameters, vorzugsweise beinhaltend die Dialysedosierung, nach einer Vielzahl von bestimmten Zeitinkrementen,
   Integrieren der Vielzahl von Werten zur Vielzahl von Zeitinkrementen, um eine integrierte Messung eines Gesamtwertes des gelieferten bedeutenden Parameters darzustellen, vorzugsweise die gelieferte Gesamtdialysedosierung, wie sie mit einem Integral oder Gesamtbetrag der Vielzahl von bestimmten Zeitinkrementen im Verhältnis ist.

8. Steuerung (12) nach Anspruch 6, in der die Steuerung (12) programmiert ist zum Berechnen einer restlichen Behandlungszeit durch Subtrahieren der Messung einer gelieferten Dialysedosierung vom vorgegebenen Dialysedosierungswert und Dividieren des erhaltenen Unterschieds durch einen durchschnittlichen Dialysanzwert, der von der Dialysedosierung dividiert durch das Zeitinkrement dargestellt ist.

9. Steuerung (12) nach Anspruch 6, in der die Steuerung (12) programmiert ist zum Berechnen einer restlichen Behandlungszeit durch Subtrahieren der Messung einer gelieferten Dialysedosierung vom vorgegebenen Dialysedosierungswert und Dividieren des erhaltenen Unterschieds durch einen augenblicklichen Dialysanzwert, der am Ende des bestimmten Zeitinkrements gemessen wird, wie er durch (KTp - KTt)/DTt dargestellt ist.

10. Steuerung nach Anspruch 1, wobei die Vorrichtung eine regelbare Ultrafiltrationspumpe (34) beinhaltet, wobei die einen oder mehreren Ausgangssteuersignale in Reaktion auf einen oder mehrere miteinander verbundene Werte, die von der Steuerung (12) erzeugt werden, zum automatischen Steuern der Geschwindigkeit der regelbaren Ultrafiltrationspumpe (22) verwendet werden.

11. Steuerung (12) nach Anspruch 9, wobei die einen oder mehreren miteinander verbundenen Werte eine multiplizierte Relation zwischen den einen oder mehreren Eingaben der Messdaten und einem Verhältnis eines Unterschieds zwischen dem vorgegebenen Dialysedosierungswert und einer Messung einer gelieferten Dialysedosierung zu einem Unterschied zwischen dem vorgegebenen Gewichtsverlustwert und einem erreichten Gewichtsverlust, oder dem Kehrwert solchen Verhältnisses, wie es von:

$$\frac{(WLp - WLTt) \cdot DTt}{(KTp - KTt)},$$

dargestellt ist, oder dem Kehrwert solcher Verhältnisse umfasst, wobei die Symbole die hier angegebene Bedeutung haben.

12. Steuerung (12) nach Anspruch 11, wobei die Steuerung (12) programmiert ist zum Erzeugen eines Steuersignals in Reaktion auf die multiplizierte Relation zum automatischen Steuern der Geschwindigkeit der regelbaren Ultrafiltrationspumpe (22), um das Verhältnis oder dessen Kehrwert aufrechtzuerhalten, wobei der eingegebene vorgegebene Gesamtgewichtsverlustbezugswert im Wesentlichen gleichzeitig mit der Lieferung des eingegebenen vorgegebenen Dialysedosierungsbezugswert erreicht werden kann.

13. Steuerung (12) nach Anspruch 10, wobei die Steuerung (12) programmiert ist zum Darstellen einer Messung eines Gesamtgewichtsverlusts, der als Funktion der Geschwindigkeit der regelbaren Ultrafiltrationspumpe (22) und eines bestimmten Zeitinkrements während einer Hämodialysebehandlung erreicht wird.

14. Steuerung (12) nach Anspruch 6, wobei die Vorrichtung (10) eine regelbare Ultrafiltrationspumpe (22) umfasst, in der die Steuerung (12) programmiert ist zum Erzeugen eines Steuersignals, um die Geschwindigkeit der regelbaren Ultrafiltrationspumpe (22) als Funktion der berechneten Behandlungszeit oder restlichen Behandlungszeit und des in die Steuerung (12) eingegebenen vorgegebenen Gewichtsverlustbezugswert automatisch zu steuern.

15. Blutbehandlungsvorrichtung (10) umfassend mindestens eine Behandlungseinheit (14), die eine semipermeablen Membran (20) beinhaltet, die die Behandlungseinheit (14) in ein erstes Abteil (16) zur Blutzirkulation und in ein zweites Abteil (18) zur Zirkulation einer Behandlungsflüssigkeit trennt, und eine Steuerung (12) nach einem der vorhergehenden Ansprüche.

16. Vorrichtung (10) nach Anspruch 15, umfassend Messmittel zum Erhalten einer oder mehrerer Messungen von Daten während einer Hämodialyse, wobei die Daten eine oder mehrere Messungen von Dialysedosierungswerten oder Dialysancewerten der Behandlungseinheit (14) darstellen können, wobei die letztere einen Hämodialysator umfasst, wobei die Vorrichtung weiter Eingabemittel zur Eingabe solcher Messdaten in die Steuerung umfasst.

17. Vorrichtung (10) nach Anspruch 16, wobei die Messmittel einen abwärts gelegenen Leitfähigkeitssensor (38) zur Messung der Dialysatleitfähigkeit in einer Dialysatleitung (32) abwärts von der Behandlungseinheit (14) umfassen.

18. Vorrichtung (10) nach Anspruch 16, wobei die Messmittel weiter einen aufwärts gelegenen Leitfähigkeitssensor (36) zur Messung der Dialysatleitfähigkeit in einer Dialysatleitung (30) aufwärts von der Behandlungseinheit (14) umfassen.

19. Vorrichtung (10) nach Anspruch 16, wobei die Steuerung (12) programmiert ist zum Darstellen eines oder mehrerer Dialysanzwerte des Hämodialysator (14) bei einem oder mehreren bestimmten Zeitinkrementen während einer Hämodialysebehandlung, und zum Berechnen einer Hämodialysebehandlungszeit oder restlichen Hämodialysebehandlungszeit durch Verbinden solcher Dialysancewerte und bestimmten Zeitinkremente mit dem vorgegebenen Dialysedosierungsbezugswert, der in die Steuerung (12) eingegeben wurde.

20. Vorrichtung (10) nach Anspruch 19, umfassend eine regelbare Ultrafiltrationspumpe (34), in der die Steuerung (12) programmiert ist zum Erzeugen eines Steuersignals zur automatischen Steuerung der Geschwindigkeit der regelbaren Ultrafiltrationspumpe (34) als Funktion der berechneten Hämodialysebehandlungszeit oder restlichen Hämodialysebehandlungszeit und des vorgegebenen Gewichtsverlustbezugswerts, der in die Steuerung (12) eingegeben wurde.

21. Vorrichtung (10) nach Anspruch 19, in der eine vorgegebene maximale Hämodialysebehandlungszeit in die Steuerung eingegeben wird, und in der die Steuerung programmiert ist zum Vergleichen der berechneten Hämodialysebehandlungszeit oder restlichen Hämodialysebehandlungszeit mit der vorgegebenen maximalen Hämodialysebehandlungszeit und, wenn eine berechnete Gesamthämodialysebehandlungszeit die vorgegebene maximale Behandlungszeit überschreitet, zum Erzeugen eines Ausgangssteuersignals zur Steuerung der Geschwindigkeit der Ultrafiltrationspumpe (34), um den vorgegebenen Gewichtsverlustbezugswert zu erreichen, wenn die vorgegebene maximale Hämodialysebehandlungszeit erreicht wird.

22. Vorrichtung (10) nach Anspruch 17, in der die Steuerung (12) mit einer Alarmvorrichtung verbunden ist, in der eine vorgegebene maximale Hämodialysebehandlungszeit in die Steuerung (12) eingegeben wird, und wobei die Steuerung (12) programmiert ist zum Vergleichen der berechneten Hämodialysebehandlungszeit oder restlichen Hämodialysebehandlungszeit mit der vorgegebenen maximalen Hämodialysebehandlungszeit und, wenn eine berechnete Gesamthämodialysebehandlungszeit die vorgegebene maximale Behandlungszeit überschreitet, zum Erzeugen eines Steuersignals zur Aktivierung der Alarmvorrichtung.

23. Vorrichtung (10) nach Anspruch 15, wobei die Steuerung (12) mit einem Anzeigebildschirm (50) verbunden ist, zur Anzeige eines Ausgangssignals der Steuerung (12), das die Messungen von gelieferten Dialysedosierungen oder Dialysanzwerten eines Hämodialysators darstellt.

24. Vorrichtung (10) nach Anspruch 15, in der die Steuerung (12) mit einem Anzeigebildschirm (50) verbunden ist, zur Anzeige eines Ausgangssignals der Steuerung (12), das die gelieferte Dialysedosierung in Reaktion auf die von der Steuerung (12) empfangenen Messdaten darstellt.

25. Vorrichtung (10) nach Anspruch 15, in der die Steuerung (12) mit einem Anzeigebildschirm (50) verbunden ist, zur Anzeige eines Ausgangssignals von der Steuerung (12), das die Hämodialysebehandlungszeit oder restliche Hämodialysebehandlungszeit, die von der Steuerung (12) berechnet wurde, darstellt.

26. Vorrichtung (10) nach Anspruch 15, in der die Steuerung (12) mit einem Anzeigebildschirm (50) verbunden ist, zur Anzeige des vorgegebenen Dialysedosierungsbezugswertes, der in die Steuerung (12) eingegeben wurde.

27. Vorrichtung (10) nach Anspruch 15, in der die Steuerung (12) mit einem Anzeigebildschirm (50) verbunden ist, zur Anzeige des vorgegebenen Gewichtsverlustbezugswertes, der in die Steuerung (12) eingegeben wurde.

28. Vorrichtung (10) nach Anspruch 15, in der die Steuerung (12) mit einem Anzeigebildschirm (50) verbunden ist, zur Anzeige der Messung eines erreichten Gesamtgewichtsverlusts.

**29.** Vorrichtung (10) nach Anspruch 15, in der die Steuerung (12) mit einem Anzeigebildschirm (50) verbunden ist, zur Anzeige der vorgegebenen maximalen Hämodialysebehandlungszeit, die in die Steuerung (12) eingegeben wurde.

**30.** Programmspeichermittel beinhaltend ein Programm für eine programmierbare Steuerung (12) nach einem der vorhergehenden Ansprüche, wobei das Programm, wenn es durch die Steuerung (12) ausgeführt wird, die Steuerung (12) ermöglicht, ein Verfahren zum automatischen Steuerung einer oder mehrerer regelbaren Operationen auszuführen, die von einer Hämodialyseüberwachungsvorrichtung (10) durchgeführt werden, umfassend mindestens eine Behandlungseinheit (14) mit einer semipermeablen Membran, die die Behandlungseinheit in ein erstes Abteil (16) zur Blutzirkulation und in ein zweites Abteil (18) zur Zirkulation einer Behandlungsflüssigkeit trennt, wobei die Vorrichtung (10) eine programmierbare Steuerung (12) umfasst oder damit verbunden ist, wobei das Verfahren die folgenden Schritte umfasst:

- Empfangen einer oder mehrerer Eingaben von während einer Behandlung gemessenen Messdaten, wobei die Messdaten ausgewählt werden aus der Gruppe umfassend die Leitfähigkeit der Behandlungsflüssigkeit abwärts von dem Dialysator (14); die Konzentration eines Stoffs abwärts von dem Dialysator (14);
- Berechnen, ausgehend von den Messdaten, mindestens eines bedeutenden Parameters, der den Verlauf einer extrakorporalen, durch die Vorrichtung (10) durchgeführten Blutbehandlung angibt,
- Vergleichen des bedeutenden Parameters mit einem vorgegebenen Bezugswert für denselben Parameter,
- Erzeugen mindestens eines Ausgangssteuersignals in Reaktion auf den Vergleich,
- automatische Steuerung, ausgehend vom Ausgangssteuersignal, einer Fluidentfernungsrate aus dem zweiten Abteil (18);

wobei der bedeutende, den Verlauf angebende Parameter ausgewählt wird aus der Gruppe umfassend:

- die Konzentration Cb eines Stoffs im Blut eines Patienten, der einer Behandlung unterworfen ist;
- die Dialysedosis KTt nach einer Zeit Tt.

**31.** Programmspeichermittel nach Anspruch 30, umfassend einen optischen Datenträger und/oder einen magnetischen Datenträger und/oder einen flüchtigen Speicherträger.

**Revendications**

**1.** Contrôleur (12) pour un appareil de traitement du sang (10), ledit appareil comprenant au moins une unité de traitement (14) incluant une membrane semi-perméable qui partage l'unité de traitement dans un premier compartiment (16) pour la circulation du sang et dans un deuxième compartiment (18) pour la circulation d'un liquide de traitement, le contrôleur (12) étant apte à:

- recevoir une ou plusieurs entrées d'informations mesurées pendant un traitement, lesdites informations mesurées étant choisies dans le groupe comprenant la conductivité du liquide de traitement en aval de l'unité de traitement (14); la concentration d'une substance dans le liquide de traitement en aval de l'unité de traitement (14);
- calculer à partir desdites données de mesure au moins un paramètre significatif indiquant le progrès d'un traitement extracorporel du sang effectué par l'appareil (10),
- comparer ledit paramètre significatif calculé avec une valeur de référence donnée pour le même paramètre,
- générer au moins un signal de commande de sortie en réponse à ladite comparaison, où

ledit signal de commande de sortie est généré pour contrôler automatiquement un taux d'élimination de fluide dudit deuxième compartiment (18);
le paramètre significatif indiquant le progrès est choisi dans le groupe comprenant:

- la concentration Cb d'une substance dans le sang d'un patient soumis à un traitement;
- la dose de dialyse KTt après un temps Tt.

**2.** Contrôleur (12) selon la revendication 1, programmé pour mettre en relation lesdits un ou plusieurs paramètres significatifs avec une valeur de référence donnée pour le dosage de dialyse et avec une valeur de référence donnée pour la perte de poids, enregistrées dans le contrôleur (12) afin d'obtenir une ou plusieurs valeurs interdépendantes et pour générer au moins un ou plusieurs signaux de commande de sortie en réponse auxdites une ou plusieurs valeurs interdépendantes pour contrôler automatiquement une ou plusieurs opérations variables effectuées par l'appareil (10).

**3.** Contrôleur (12) selon la revendication 2, où lesdites une ou plusieurs valeurs interdépendantes comprennent une relation multipliée entre lesdites une ou plusieurs entrées desdites données de mesure et un rapport entre une différence entre ladite valeur de dosage de dialyse donnée et une mesure d'un dosage de dialyse délivré et une différence entre ladite valeur de perte de poids donnée et une perte de poids atteinte, ou l'inverse de ce rapport représenté par:

$$\frac{(WL_p - WL_{Tt}) \cdot DT_t}{(KT_p - KT_t)},$$

ou l'inverse desdits rapports, où les symboles ont la signification indiquée ici.

4. Contrôleur (12) selon la revendication 1, où le contrôleur (12) est programmé pour calculer un dosage de dialyse délivré total en réponse aux donnée de mesure reçues par le contrôleur (12), et pour générer un signal de commande de sortie lorsque ledit donnée de mesure reçue par le contrôleur (12) réfléchit une mesure d'un dosage de dialyse délivré total qui approche ou égale ladite valeur de dosage de dialyse donnée.

5. Contrôleur (12) selon la revendication 1, où le contrôleur (12) est programmé pour calculer un dosage de dialyse délivré total à un ou plusieurs incréments de temps déterminés pendant un traitement en réponse aux données de mesure reçues par le contrôleur auxdits un ou plusieurs incréments de temps déterminés, et pour générer un signal de commande de sortie lorsque ledit donnée de mesure reçues par le contrôleur (12) réfléchit une mesure d'un dosage de dialyse délivré total qui approche ou égale ladite valeur de dosage de dialyse donnée.

6. Contrôleur (12) selon la revendication 1, où le contrôleur (12) est programmé pour calculer un temps de traitement d'hémodialyse ou un temps restant de traitement d'hémodialyse en mettant en relation un calcul d'un dosage de dialyse délivré réfléchi par une entrée de données de mesure reçues par le contrôleur (12) après un incrément de temps déterminé pendant un traitement d'hémodialyse avec ladite valeur de dosage de dialyse donnée.

7. Contrôleur (12) selon la revendication 6, où le contrôleur est programmé pour:

déterminer une pluralité de valeurs dudit paramètre significatif, comprenant de préférence le dosage de dialyse, après une pluralité d'incréments de temps déterminés,
intégrer ladite pluralité de valeurs sur ladite pluralité d'incréments de temps pour réfléchir une mesure intégrée d'une valeur totale dudit paramètre significatif délivré, de préférence le dosage de dialyse délivré total, mis en relation avec un intégral ou total de ladite pluralité d'incréments de temps déterminés.

8. Contrôleur (12) selon la revendication 6, où le contrôleur (12) est programmé pour calculer un temps de traitement restant en soustrayant ladite mesure d'un dosage de dialyse délivré de ladite valeur de dosage de dialyse donnée et en divisant la différence obtenue par une valeur de dialysance moyenne représentée par ledit dosage de dialyse délivré divisé par ledit incrément de temps déterminé.

9. Contrôleur (12) selon la revendication 6, où le contrôleur (12) est programmé pour calculer un temps de traitement restant en soustrayant ladite mesure d'un dosage de dialyse délivré de ladite valeur de dosage de dialyse donnée et en divisant la différence obtenue par une valeur de dialysance instantanée mesurée à la fin dudit incrément de temps déterminé, représenté par $(KT_p - KT_t)/DT_t$.

10. Contrôleur selon la revendication 1, où l'appareil comprend une pompe d'ultrafiltration à vitesse variable (34), lesdits un ou plusieurs signaux de commande de sortie en réponse auxdites une ou plusieurs valeurs interdépendantes générées par le contrôleur (12) étant utilisés pour contrôler automatiquement la vitesse de ladite pompe d'ultrafiltration à vitesse variable (22).

11. Contrôleur (12) selon la revendication 9, où lesdites une ou plusieurs valeurs interdépendantes comprennent une relation multipliée entre lesdites une ou plusieurs entrées desdites données de mesure et un rapport entre une différence entre ladite valeur de dosage de dialyse donnée et une mesure d'un dosage de dialyse délivré et une différence entre ladite valeur de perte de poids donnée et une perte de poids atteinte, ou l'inverse de ce rapport représenté par:

$$\frac{(WL_p - WL_{Tt}) \cdot DT_t}{(KT_p - KT_t)},$$

ou l'inverse desdits rapports, où les symboles ont la signification indiquée ici.

12. Contrôleur (12) selon la revendication 11, où le contrôleur (12) est programmé pour générer un signal de commande en réponse à ladite relation multipliée afin de contrôler automatiquement la vitesse de ladite pompe d'ultrafiltration à vitesse variable (22) pour maintenir ledit rapport ou son inverse, la valeur de référence introduite pour la perte de poids totale donnée pouvant être obtenue substantiellement au même temps que la livraison de la valeur de référence introduite pour le dosage de dialyse donné.

**13.** Contrôleur (12) selon la revendication 10, où le contrôleur (12) est programmé pour réfléchir une mesure d'une perte de poids totale obtenue en fonction de la vitesse de la pompe d'ultrafiltration à vitesse variable (22) et d'un incrément de temps déterminé pendant un traitement d'hémodialyse.

**14.** Contrôleur (12) selon la revendication 6, l'appareil (10) comprenant une pompe d'ultrafiltration à vitesse variable (22), où le contrôleur (12) est programmé pour générer un signal de commande afin de contrôler automatiquement la vitesse de ladite pompe d'ultrafiltration à vitesse variable (22) en fonction dudit temps de traitement calculé ou dudit temps de traitement restant et de ladite valeur de référence donnée pour la perte de poids introduite dans le contrôleur (12).

**15.** Appareil de traitement du sang (10) comprenant au moins une unité de traitement (14) incluant une membrane semi-perméable (20) qui partage l'unité de traitement (14) dans un premier compartiment (16) pour la circulation du sang et dans un deuxième compartiment (18) pour la circulation d'un liquide de traitement, et un contrôleur (12) selon une quelconque des revendications précédentes.

**16.** Appareil (10) selon la revendication 15, comprenant des moyens de mesure pour obtenir une ou plusieurs mesures de données pendant un traitement d'hémodialyse, lesquelles données peuvent réfléchir une ou plusieurs mesures de valeurs de dosage de dialyse ou valeurs de dialysance de ladite unité de traitement (14), cette dernière comprenant un hémodialyseur, l'appareil comprenant aussi des moyens d'entrée pour introduire lesdites données mesurées dans le contrôleur.

**17.** Appareil (10) selon la revendication 16, où les moyens de mesure comprennent un capteur de conductivité en aval (38) pour mesurer la conductivité du dialysat dans un conduit de dialysat (32) en aval de l'unité de traitement (14).

**18.** Appareil (10) selon la revendication 16, où les moyens de mesure comprennent en plus un capteur de conductivité en amont (36) pour mesurer la conductivité du dialysat dans un conduit de dialysat (30) en amont de l'unité de traitement (14).

**19.** Appareil (10) selon la revendication 16, où le contrôleur (12) est programmé pour réfléchir une ou plusieurs valeurs de dialysance dudit hémodialyseur (14) à un ou plusieurs incréments de temps déterminés pendant un traitement d'hémodialyse et pour calculer un temps de traitement d'hémodialyse ou un temps de traitement d'hémodialyse restant en mettant en relation lesdites valeurs de dialysance et lesdits incréments de temps déterminés avec ladite valeur de référence donnée pour le dosage de dialyse introduite dans le contrôleur (12).

**20.** Appareil (10) selon la revendication 19, comprenant une pompe d'ultrafiltration à vitesse variable (34), où le contrôleur (12) est programmé pour générer un signal de commande afin de contrôler automatiquement la vitesse de la pompe d'ultrafiltration à vitesse variable (34) en fonction dudit temps de traitement d'hémodialyse calculé ou dudit temps de traitement d'hémodialyse restant et de ladite valeur de référence donnée pour la perte de poids introduite dans le contrôleur (12).

**21.** Appareil (10) selon la revendication 19, où un temps de traitement d'hémodialyse maximum donné est introduit dans le contrôleur et où le contrôleur est programmé pour comparer ledit temps de traitement d'hémodialyse calculé ou temps de traitement d'hémodialyse restant avec ledit temps de traitement d'hémodialyse maximum donné et, si un temps de traitement d'hémodialyse donné total dépasse ledit temps de traitement maximum donné, pour générer un signal de commande de sortie pour contrôler la vitesse de la pompe d'ultrafiltration (34) afin d'atteindre ladite valeur de référence donnée pour la perte de poids lorsque ledit temps de traitement d'hémodialyse maximum donné est atteint.

**22.** Appareil (10) selon la revendication 17, où le contrôleur (12) est associé à un dispositif d'alarme, où un temps de traitement d'hémodialyse maximum donné est introduit dans le contrôleur (12) et où le contrôleur (12) est programmé pour comparer ledit temps de traitement d'hémodialyse calculé ou temps de traitement d'hémodialyse restant avec ledit temps de traitement d'hémodialyse maximum donné et, si un temps de traitement d'hémodialyse calculé total dépasse ledit temps de traitement maximum donné introduit, pour générer un signal de commande pour activer ledit dispositif d'alarme.

**23.** Appareil (10) selon la revendication 15, où le contrôleur (12) est associé à un écran de visualisation (50) apte à visualiser un signal de sortie du contrôleur (12) qui réfléchit lesdites mesures de dosages de dialyse délivrés ou valeurs de dialysance d'un hémodialyseur réfléchies par lesdites données de mesure.

**24.** Appareil (10) selon la revendication 15, où le contrôleur (12) est associé à un écran de visualisation (50) apte à visualiser un signal de sortie du contrôleur (12) qui réfléchit ledit dosage de dialyse délivré total réfléchi en réponse aux données de mesure reçues par le contrôleur (12).

25. Appareil (10) selon la revendication 15, où le contrôleur (12) est associé à un écran de visualisation (50) apte à visualiser un signal de sortie du contrôleur (12) qui réfléchit ledit temps de traitement d'hémodialyse ou temps de traitement d'hémodialyse restant calculé par le contrôleur (12).

26. Appareil (10) selon la revendication 15, où le contrôleur (12) est associé à un écran de visualisation (50) apte à visualiser ladite valeur de référence donnée pour le dosage de dialyse introduite dans le contrôleur (12).

27. Appareil (10) selon la revendication 15, où le contrôleur (12) est associé à un écran de visualisation (50) apte à visualiser ladite valeur de référence donnée pour la perte de poids introduite dans le contrôleur (12).

28. Appareil (10) selon la revendication 15, où le contrôleur (12) est associé à un écran de visualisation (50) apte à visualiser ladite mesure d'une perte de poids totale atteinte.

29. Appareil (10) selon la revendication 15, où le contrôleur (12) est associé à un écran de visualisation (50) apte à visualiser ledit temps de traitement d'hémodialyse maximum donné introduit dans le contrôleur (12).

30. Moyen de mémorisation de programmes comprenant un programme pour un contrôleur programmable (12) selon une des revendications précédentes, le programme, une fois exécuté par le contrôleur (12), rendant le contrôleur (12) apte à exécuter un procédé pour commander automatiquement une ou plusieurs opérations variables effectuées par un appareil de surveillance de l'hémodialyse (10) comprenant au moins une unité de traitement (14) incluant une membrane semi-perméable qui partage l'unité de traitement dans un premier compartiment (16) pour la circulation du sang et dans un deuxième compartiment (18) pour la circulation d'un liquide de traitement, ledit appareil (10) étant associé à ou comprenant le contrôleur programmable (12), ledit procédé incluant les étapes suivantes:

  - recevoir une ou plusieurs entrées de données de mesure mesurées pendant un traitement, lesdites données de mesure étant une choisies dans le groupe comprenant la conductivité du liquide de traitement en aval du dialyseur (14); la concentration d'une substance dans le liquide de traitement en aval du dialyseur (14);
  - calculer à partir desdites données de mesure au moins un paramètre significatif indiquant le progrès d'un traitement extracorporel du sang effectué par l'appareil (10),
  - comparer ledit paramètre significatif calculé avec une valeur de référence donnée pour le même paramètre,
  - générer au moins un signal de commande de sortie en réponse à ladite comparaison,
  - commander automatiquement à partir dudit signal de contrôle de sortie un taux d'élimination de fluide dudit deuxième compartiment (18).

où le paramètre significatif indiquant le progrès est choisi dans le groupe comprenant:

  - la concentration Cb d'une substance dans le sang d'un patient soumis à un traitement;
  - la dose de dialyse KTt après un temps Tt.

31. Moyen de mémorisation de programmes selon la revendication 30, comprenant un support de données optique et/ou un support de données magnétique et/ou un support à mémoire volatile.

Fig 1

EP 1 396 274 B2

# Dialysis delivery control overview

Dialysance measure DTt at time Tt

positive

If Tt+Ttr > Tm

Red light flashes

Staff intervention

Estimation of KTt, the integrated KT Achieved by time Tt.

Management of low dialysance

negative

Normal cycle of automatic UF control

Estimation of dialysis remaining time Ttr

If Ttr < 15 min

Calculation of the UF rate UFTt=(WLp-WLTt)/Ttr

dialysis remaining time set at Tm-Tt

End of dialysis session after failure of staff intervention

Set the UF pump at UFTt

UF rate set at (WLp-WLTt)/(Tm-Tt)

Calculation of the UF rate UFTt = (WLp-WLTt) / Ttr

End of dialysis session in normal case

Orange light flashes

WLp achieved

Set the UF pump at UFTt

dPp achieved

Green light flashes

Patient can be disconnected

Green light flashes

Fig 2

Patient can be disconnected

EP 1 396 274 B2

# Dialysis delivery control
## User interface

50

| red | orange | green |
|---|---|---|
| Flashes when the last dialysance does not allow to complete prescribed KT within the maximum acceptable Dialysis time | Flashes 15' before prescribed KT and total weight loss are completed | Flashes when prescribed KT and total weight loss are completed |

52 ◯ Temporary by-pass    54 ◯ Permanent by-pass

Maximum acceptable dialysis time

Dialysis time forcasted to achieve prescribed KT

| Present dialysis time | Forcasted remaining dialysis time | 2h25min |
|---|---|---|

Name: xxxx.. KT: 42 liters  TWL: 3.6 kg  Max dialysis time: 4h15min

## Fig 3

EP 1 396 274 B2

# Normal cycle of automatic UF control

Before the start of the session, total weight loss (TWLp), KT to be acheived (KTp), and the maximum acceptable dialysis time (Tm) are entered by the doctor.

Dialysance
measure DTt
At time Tt (1)

During the session, at any time t:
Dialysance measure based on conductivity measures by probes c1 and c2 at dialysate inlet and dialysate outlet. (1)
(see Fig1)

Estimation of KTt,
the integrated KT
Achieved by time Tt.(2)

Normal cycle
of automatic
UF control

Estimation of KTt, the integrated KT achieved up to time t. (2)

Estimation of
dialysis
remaining time Ttr (3)

Estimation of dialysis remaining time Ttr, necessary to acheive prescribed KTp: (3)

$$Ttr = (KTp\text{-}KTt) / DTt$$

Calculation of
the UF rate
UFTt = (WLp-WLTt) / Ttr (4)

Calculation of the UF rate needed to acheive the total weight loss during the remaining time Ttr (4)

$$UFTt = (WLp\text{-}WLTt) / Ttr$$

Set the UF pump
at UFTt (5)

Set the UF pump at UFTt (5)

# Fig 4

EP 1 396 274 B2

## End of dialysis session in normal case

If Ttr < 15 min

Calculation of
the UF rate
UFTt = (WLp-WLTt)/ Ttr

Set the UF pump
At UFTt

Orange light flashes

WLp achieved

Green light flashes

Patient can be
dlconnected

**Fig 5**

When the estimated remaining dialysis time <15 min, no further dialysance measures are made.

The UF rate is updated for the last time,

The orange light flashes indicating that the patient can be disconnected in 15 min.

When the prescribed total weight loss (WLp) is achieved, the green light flashes indicating that both the prescribed total weight loss and the prescribed dialysis dose (KTp) have been achieved .

The patient can be disconnected.

# Management of low dialysance

If Tt+Ttr > Tm

Red light flashes

Staff Intervention

positive

Normal cycle of automatic UF control

negative

End of dialysis session after failure of staff intervention

**Fig 6**

A low dialysance is measured and the calculated remaining time(Ttr) needed to achieve prescribed Kt leads to a total time which is greater than the maximum acceptable time.

Red light.flashes.

Attending staff is thereby invited to check the dialysis setting to detect and to correct possible causes of low dialysance.

The temporary by-pass button is pressed in order to cancel the flashing light.

If the correction made is succesful, automatic management of UF rate is restarted.

If not, the system moves to appropriate procedure with fixed UF rate. In this case, it is unlikely that the prescribed KTp will be achieved.

EP 1 396 274 B2

## End of dialysis session after failure of staff intervention

negative

dialysis remaining time set at Tm-Tt

UF rate set at (WLp-WLTt)/ (Tm-Tt)

dPp achieved

Green light flashes

Patient can be disconnected

If the correction attempt fails,

The permanent by-pass key is pressed

The dialysis remaining time is set so that total dialysis time equals maximum acceptable dialysis time.

UF rate is set in order to achieve the total weight loss at the maximum acceptable dialysis time.

When the total weight loss is achieved, the green light flashes indicating that the weight loss has been achieved .

The patient can be disconnected.

## Fig 7

EP 1 396 274 B2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0330892 A, Gambro
- EP 0495412 A
- WO 9855166 A
- EP 0547025 B1
- EP 0658352 B1